# FASCICULE DE BREVET EUROPEEN

(11) **EP 1 520 561 B1**
(45) Date de publication et mention de la délivrance du brevet: **27.01.2010**
(21) Numéro de dépôt: 04300619.6
(22) Date de dépôt: 23.09.2004
(51) Int. Cl.: A61F 2/40, A61B 17/86

(54) **Prothèse de cavité glénoide de la scapula**
Schulterblatt-Gelenkpfannenprothese
Scapular glenoid cavity prosthesis

(30) Priorité: 01.10.2003 FR 0311511
(43) Date de publication de la demande: 06.04.2005
(73) Titulaire: Biomet France, 26000 Valence (FR); Beguin, Laurent, 69390 Charly (FR); Bouttens, Denis, 62170 Saint-Josse (FR); Cohn, Charles, 83600 Les Adrets de l'Esterel (FR); Declercq, Geert, 2970 Schilde (BE); Grimberg, Jean, 78810 Feucherolles (FR); Huguet, Dominique, 44700 Orvault (FR); Lesprit, Eric, 33000 Bordeaux (FR); Massart, Pierre, 38530 Chapareillan (FR); Rio, Bruno, 54000 Nancy (FR); Teissier, Jacques, 34170 Castelnau Le Lez (FR); Toussaint, Bruno, 74940 Annecy le Vieux (FR); Zipoli, Bruno, 40100 Dax (FR)
(72) Inventeur: Beguin, Laurent, 69390 Charly (FR); Brunnarius, Yann, 07130 Saint Péray (FR); Bouttens, Denis, 62170 Saint Josse (FR); Cohn, Charles, 83600 Les Adrets de l'Esterel (FR); Declercq, Geert, 2970 Schilde (BE); Grimberg, Jean, 78810 Feucherolles (FR); Huguet, Dominique, 44700 Orvault (FR); Lesprit, Eric, 33000 Bordeaux (FR); Massart, Pierre, 38530 Chapareillan (FR); Rio, Bruno, 54000 Nancy (FR); Teissier, Jacques, 34170 Castelnau Le Lez (FR); Toussaint, Bruno, 74940 Annecy Le Vieux (FR); Zipoli, Bruno, 40100 Dax (FR)
(74) Mandataire: Blanchard, Eugène Gilles

(56) Documents cités:
- EP-A- 0 715 836
- EP-A- 1 336 396
- WO-A-01/47442
- FR-A- 2 652 498
- FR-A- 2 683 142
- FR-A- 2 695 313
- FR-A- 2 704 747
- FR-A- 2 737 107
- US-A- 4 550 450
- US-A- 5 888 204
- US-A1- 2003 055 507

## Description

L'invention concerne une prothèse de la cavité glénoïde de la scapula, et se rattache de manière générale aux prothèses d'épaule.

En fonction de la nature de la pathologie dont peut faire l'objet l'épaule d'un patient (douleurs, arthrose, polyarthrite, problèmes tendineux ou musculaires, traumatismes, etc .), il est quelque fois requis, notamment lorsque la cavité glénoïde a été détruite partiellement ou en totalité, mais également lorsque l'on observe une rupture de la coiffe des rotateurs, la nécessité de la mise en place d'une telle prothèse de la cavité glénoïde.

Traditionnellement, cette prothèse se présente sous la forme d'une embase venant se fixer au niveau de ladite cavité glénoïde de la scapula, tout au moins de ce qu'il en reste. Une telle cavité glénoïde est par exemple décrite dans le document FR-A-2 683 142.

Les prothèses de cavité glénoïde connues à ce jour sont destinées à recevoir un insert destiné à coopérer avec la tête humérale ou avec un implant huméral, et se présentent donc sous la forme d'une concavité de forme sensiblement complémentaire généralement non congruente avec ladite tête humérale ou avec ledit implant huméral. En d'autres termes, l'insert est généralement une pièce femelle.

Il peut cependant être quelques fois requis, en fonction de la pathologie observée, de mettre en oeuvre non pas une pièce femelle, mais une pièce mâle se présentant alors sous la forme d'une tête sphérique ou sensiblement sphérique fixée sur l'embase de la prothèse de la cavité glénoïde. Dans cette hypothèse, il est donc généralement nécessaire de faire appel à une autre prothèse de cavité glénoïde pour permettre la mise en oeuvre d'un tel insert mâle.

En d'autres termes, la prothèse en question ne présente pas le caractère modulaire souhaitable pour les praticiens, mais également sur le plan économique.

On a décrit dans le document FR-A-2 652 498 une prothèse totale d'épaule présentant un certain caractère de modularité. Dans ce document, tant l'implant huméral que la prothèse de la cavité glénoïde comportent des éléments complémentaires conférant ce caractère modulable. S'agissant de la prothèse de la cavité glénoïde, ces éléments sont fixés à la périphérie de l'embase, notamment par coopération d'un filetage, respectivement interne pour l'insert et externe pour l'embase. Cependant, ce mode de fixation se révèle à l'usage, non satisfaisant, de par les risques de micro-déplacements susceptibles d'intervenir, induisant de fait des risques de descellement de la prothèse de la cavité glénoïde. Parallèlement, lors des mouvements de l'articulation considérée, on observe une usure de l'os de la cavité glénoïde au niveau de laquelle est implantée ladite prothèse inhérente à l'implant, et se traduisant par une rupture des moyens de fixation de l'embase constitutif de l'implant.

L'objet de la présente invention est de proposer une prothèse de cavité glénoïde de la scapula présentant également ce caractère modulaire et s'affranchissant des inconvénients mentionnés précédemment.

Cette prothèse de la cavité glénoïde de la scapula comprend une embase, destinée à être fixée sur la cavité glénoïde du patient à prothéser au moyen de vis d'ancrage, ladite embase comportant un plateau apte à recevoir un insert femelle, et ladite embase étant apte à recevoir un insert mâle, lesdits inserts étant destinés à coopérer avec la tête humérale de l'articulation de l'épaule considérée ou avec un implant huméral mis en place au niveau de ladite articulation.

Cette prothèse se caractérise :
- en ce que la face externe du rebord périphérique dont est munie l'embase est conformée en tronc de cône morse, apte à coopérer avec un insert mâle muni d'une portée de forme complémentaire,
- et en ce que le plateau de l'embase est muni de moyens destinés à coopérer avec un insert femelle afin d'assurer sa fixation par clipsage en son sein.

Selon l'invention, l'embase est pourvue de deux perçages traversants, inclinés chacun d'une valeur angulaire comprise entre 20° et 35° par rapport au plan général du plateau, et destinés à permettre le passage de vis d'ancrage au sein de la cavité glénoïde.

Selon l'invention, la face de l'embase destinée à venir au contact de l'os de la cavité glénoïde est bombée, et est munie d'une quille destinée à être implantée au sein de ladite cavité glénoïde.

En outre, avantageusement, cette même face présente deux méplats situés en positions extrêmes par rapport à la partie bombée, et donc situés de part et d'autre de la zone bombée, destinés à prendre appui, après fraisage approprié, au sein de la cavité glénoïde. Ce faisant, ces méplats assurent une fonction stabilisatrice de la prothèse, évitant les phénomènes de basculement inhérents au fonctionnement anatomique de l'articulation, et renforçant la stabilité primaire de ladite prothèse.

En outre, et selon une autre caractéristique de l'invention, la quille prolongeant la face de l'embase destinée à venir au contact de l'os de la cavité glénoïde, est percée, de telle sorte à favoriser la fixation secondaire de la prothèse par repousse ou migration osseuse.

Selon l'invention, les moyens assurant la coopération et la fixation de l'insert femelle avec le plateau de l'embase sont constitués d'orifices non traversants propres à coopérer avec des pions de diamètre approprié issus de la partie inférieure de l'insert femelle, ou d'évidements réalisés à la périphérie interne et intérieure de l'embase, propres à coopérer avec des saillies de dimensions correspondantes issues de la base de l'insert femelle.

Selon une autre caractéristique de l'invention, les perçages traversants du plateau sont chacun munis d'une rotule à expansion, susceptible de coopérer avec les vis d'ancrage, et destinée à assurer le blocage de ladite vis selon l'orientation choisie par le praticien. Cette rotule est elle-même munie d'ergots périphériques formant saillie par rapport à son enveloppe externe, lesdits ergots étant destinés à être reçus dans des évidements correspondants, ménagés au sein d'une bague soudée au sein desdits perçages.

La manière de réaliser l'invention et les avantages qui en découlent ressortiront mieux de l'exemple de réalisation qui suit, donné à titre indicatif et non limitatif à l'appui des figures annexées.
La figure 1 est une représentation schématique en perspective de l'embase de la prothèse de la cavité glénoïde conforme à l'invention, munie de ses vis d'ancrage.
La figure 2 est une représentation en vue latérale de l'embase de la figure 1.
La figure 3 est une représentation schématique en section de l'embase de la figure 1.
La figure 4 est une représentation schématique en perspective de l'embase conforme à l'invention en version expurgée et vue du dessus.
La figure 5 est une représentation schématique en perspective de l'embase de la figure 4 vue du dessous.
La figure 6 est une vue de détail de la figure 5.
La figure 7 est une représentation schématique en perspective d'une rotule à expansion, conforme à l'invention.
La figure 8 est une représentation schématique en perspective d'une bague conforme à l'invention, destinée à coopérer avec la rotule de la figure 7, et dont la figure 9 est une vue en plan.
La figure 10 est une représentation schématique en perspective de l'embase de la prothèse de la cavité glénoïde conforme à l'invention, munie d'un insert femelle.
La figure 11 est une représentation en vue latérale de la figure 10.
La figure 12 est une représentation schématique en perspective d'une vue du dessous de l'insert femelle conforme à l'invention.
La figure 13 est une représentation schématique en perspective de l'embase de la cavité glénoïde conforme à l'invention, munie d'un insert mâle conforme à l'invention.
La figure 14 est une vue latérale de la figure 13.
La figure 15 est une représentation schématique en perspective d'une vue du dessous de l'insert mâle conforme à l'invention.

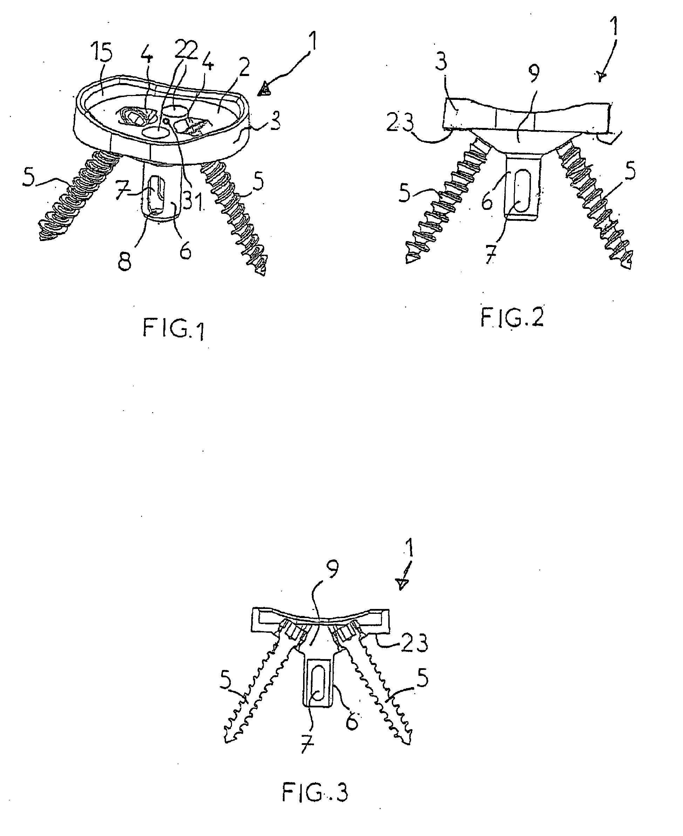

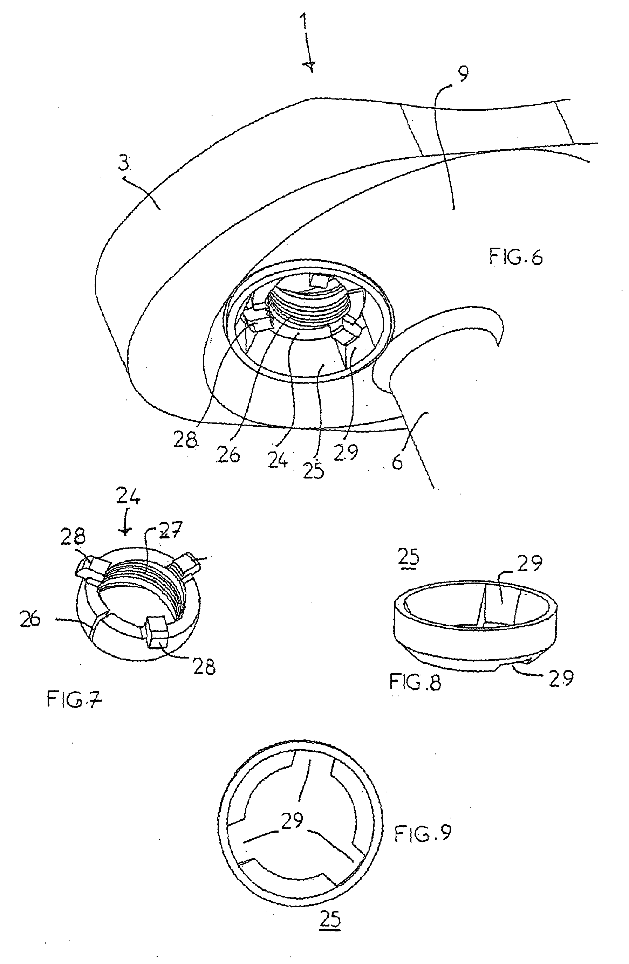

On a donc représenté en relation avec les figures 1 à 6, la prothèse de la cavité glénoïde de la scapula conforme à la présente invention. Fondamentalement, celle-ci est constituée d'une embase (1), comportant un plateau (2), destiné à être fixé par ancrage au sein de la cavité glénoïde osseuse du patient à prothéser, après préparation, et notamment fraisage par le praticien.

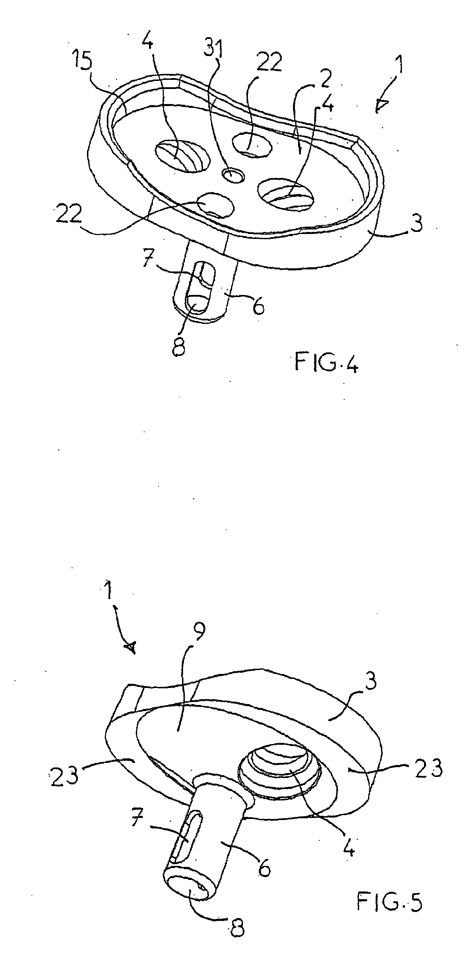

Cette embase (1) présente donc une face avant ou supérieure sensiblement plane, dénommée plateau (2) et une face inférieure destinée à venir au contact de l'os. Selon une caractéristique de l'invention, cette face inférieure est bombée (9), ainsi qu'on peut l'observer sur les figures 2 et 5. En outre, la face inférieure présente deux méplats (23), situés de part et d'autre de la forme bombée (9). Cette forme particulière contribue à conférer une certaine stabilité à la prothèse lors de sa mise en place. En effet, l'association de la forme bombée (9) avec les méplats coopérant avec des zones fraisées préalablement réalisées au sein de la cavité glénoïde osseuse, permet tout d'abord d'optimiser le contact os - implant, et en outre, d'éviter les phénomènes de basculement de l'embase, et donc de la prothèse, phénomènes inhérents au fonctionnement même de l'articulation de l'épaule.

Cette stabilité est en outre optimisée par la présence d'une quille (6), orientée sensiblement perpendiculairement par rapport à l'embase (1) et prolongeant ladite partie bombée (9). Cette quille (6) est destinée à venir se positionner dans un alésage préalablement réalisé par le praticien au sein de la cavité glénoïde osseuse.

Selon une caractéristique de l'invention, cette quille est percée d'orifices traversants, respectivement (7) le long de sa génératrice et (8) au niveau de son extrémité inférieure. Ces orifices traversants sont destinés à favoriser la tenue de la prothèse par fixation secondaire (migration et repousse osseuses).

Cette embase permet de par sa structure de conserver le plus possible le capital osseux de la cavité glénoïde.

Corollairement, l'ancrage de l'embase (1) au sein de la cavité glénoïde est réalisé au moyen de deux vis d'ancrage (5). A cet effet, le plateau (2) de l'embase est percé de deux orifices traversants inclinés (4) pour favoriser le passage des vis (5) autour d'un système de blocage décrit ci-après plus en détail.

Ces vis sont orientables de plus ou moins 12° degré par rapport à l'axe des orifices (4), afin de permettre au praticien de les placer dans le tubercule supra et infra glénoïdien.

Les orifices (4) sont inclinés d'une valeur angulaire voisine de 30° par rapport au plan contenant la face supérieure du plateau (2) de l'embase. Les vis, avantageusement réalisées en alliage de titane ou acier inoxydable, sont autoperforantes, autotaraudeuses et rétrocoupantes. L'âme conique des tiges et le filetage cylindrique dont elles sont munies favorisent l'ancrage dans l'os, et notamment, dans la cavité glénoïde considérée.

La tête de vis est conique et possède un quadruple filetage destiné à coopérer avec le système de blocage. Plus précisément, la tête de vis est munie de quatre filets parallèles, débouchant au niveau de la base inférieure de la tête selon un angle de 90° entre deux filets consécutifs. Ces quatre filets sont destinés à coopérer avec un quadruple taraudage correspondant, ménagé au niveau de la paroi interne du système de blocage, décrit ci-après plus en détail. De par ce quadruple filetage, le praticien est facilité dans sa tâche lors de l'opération de vissage, dans la mesure où le nombre de possibilités de faire mordre la vis avec le système de blocage est multiplié par seize.

Le système de blocage, dont il est fait référence précédemment, est constitué d'une rotule à expansion (24) et d'une bague (25). Cette rotule est fendue (26) le long de l'une de ses génératrices, de telle sorte à permettre son expansion. Elle possède un quadruple taraudage (27) interne, destiné à coopérer avec le quadruple filetage des têtes des vis (5). Ce faisant, le vissage de la vis dans la rotule (24) écarte cette dernière, qui se bloque alors au niveau de la bague (25) soudée au niveau de chacun des orifices (4) situés au sein de l'embase selon l'orientation souhaitée par le praticien. En outre, la base de la rotule (24) est munie de trois ergots (28), formant saillie par rapport à l'enveloppe de la rotule, périodiquement répartis, et destinés à être reçus dans des logements correspondants (29) de ladite bague (25). Ce faisant, de par la coopération entre les ergots (28) et les logements (29), la rotule (24) ne peut tourner lors de la mise en place des vis (5) par le praticien.

Selon l'invention, le plateau (2) de l'embase (1) est pourvu à sa périphérie d'un rebord (3) de forme particulière, puisqu'il est en forme de cône morse ou, plus exactement d'un tronc de cône morse. Il est destiné à coopérer avec l'insert mâle décrit ci-après plus en détail.

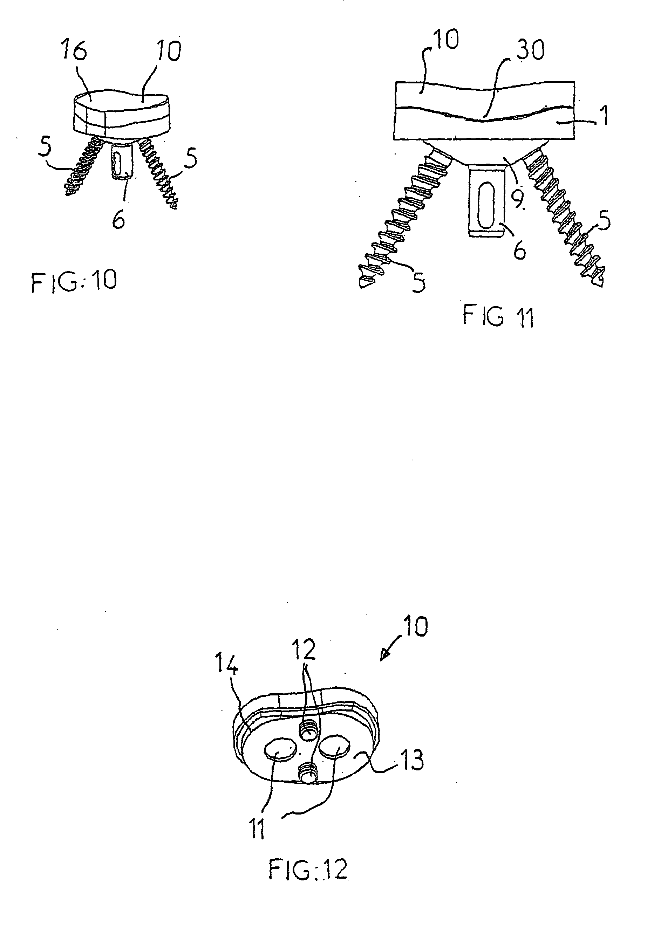

On a représenté en relation avec les figures 10 à 12 l'insert femelle (10) conforme à l'invention. Celui-ci est destiné à coopérer avec l'embase décrite précédemment. Cet insert est notamment réalisé en polyéthylène. Il présente une épaisseur minimum de 5 mm au niveau de sa zone la plus mince. Il est muni au niveau de sa face inférieure (13), de deux pions (12) de positionnement et de fixation destinés à coopérer par clipsage avec des orifices non traversants (22) ménagés au sein de plateau (1) de l'embase.

Cet insert femelle (10) vient donc s'enclipser sur ladite embase, d'une part de par la coopération des pions (12) avec les orifices (22) et, d'autre part par la coopération entre le bord périphérique interne (14) de l'insert sur la paroi interne (15) du rebord périphérique (3) de l'embase. En outre, les zones antéro-postérieures (30) de l'insert possèdent un accroissement de matière, permettant d'augmenter la longévité de l'implant. En effet, compte tenu de la sollicitation de l'insert à ce niveau inhérent au mode de fonctionnement de l'articulation, on observe généralement une plus grande usure à ce niveau. Enfin, la face inférieure (13) de l'insert est pourvue de dégagements (11) garantissant le passage des têtes de vis (5).

La face supérieure (16) de l'insert est de forme concave, et notamment anatomique, propre à coopérer avec la tête humérale ou avec un implant huméral dans le cadre de la mise en oeuvre d'une prothèse totale d'épaule.

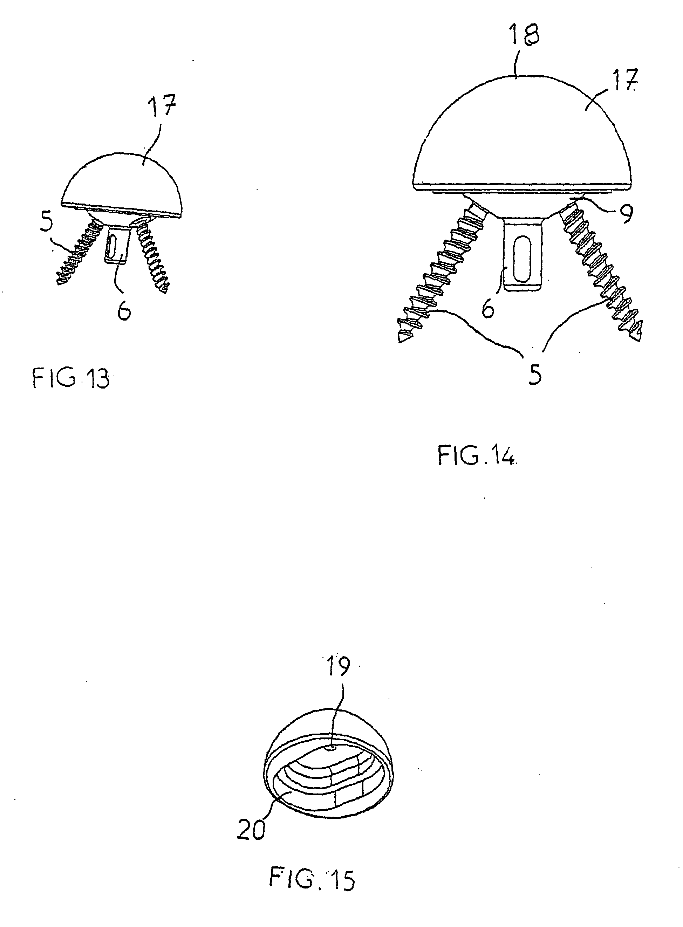

On a représenté en relation avec les figures 13 à 15, la mise en oeuvre d'un insert mâle en relation avec l'embase conforme à l'invention. Cet insert mâle (17) est de forme hémisphérique, dont le pôle (18) est percé d'un orifice (19) destiné à permettre sa mise en place par un ancillaire approprié sur l'embase (1) ou son ablation.

Cet insert mâle (17) est par exemple réalisé en acier inoxydable. Sa fixation sur l'embase est réalisée par emmanchement conique sur le cône morse ménagé à la périphérie du rebord (3) de ladite embase. Plus précisément, l'emmanchement se fait au niveau de sa paroi interne inférieure (20), comme on l'observe sur la figure 15. Cette fixation est en outre sécurisée au moyen d'une vis passant par l'orifice (19) et venant se visser dans un orifice taraudé (31) ménagé sur le plateau (2) de l'embase (1).

On conçoit tout l'intérêt de la prothèse conforme à l'invention, notamment de par son caractère modulaire, permettant au praticien, à partir d'une embase standard, la mise en oeuvre d'un insert adapté au traumatisme ou à la pathologie, ledit insert étant positionnable de manière facile et sûre évitant les inconvénients liés aux phénomènes de migration et d'instabilité, tant de l'embase que des inserts.

## Revendications

1. Prothèse de la cavité glénoïde de la scapula comprenant une embase (1), destinée à être fixée sur la cavité glénoïde du patient à prothéser au moyen de vis d'ancrage (5), ladite embase (1) comportant un plateau (2) apte à recevoir un insert mâle (17) et ladite embase étant apte à recevoir un insert femelle (10), lesdits inserts (10, 17) étant destinés à coopérer avec la tête humérale de l'articulation de l'épaule considérée ou un implant huméral mis en place au niveau de ladite articulation, ***caractérisée :***
- **en ce que** la face externe du rebord périphérique (3) dont est munie l'embase (1) est conformée en tronc de cône morse, apte à coopérer avec un insert mâle (17) muni d'une portée de forme complémentaire,
- et **en ce que** le plateau (2) de l'embase (1) est muni de moyens (22), destinés à coopérer avec un insert femelle (10) afin d'assurer sa fixation par clipsage en son sein.

2. Prothèse de la cavité glénoïde de la scapula selon la revendication 1, ***caractérisée* en ce que** le plateau (2) est pourvu de deux perçages traversants (4), inclinés chacun d'une valeur angulaire comprise entre 20° et 35° par rapport au plan général du plateau, et destinés à permettre le passage de vis d'ancrage (5) au sein de la cavité glénoïde.

3. Prothèse de la cavité glénoïde de la scapula selon l'une des revendications 1 et 2, ***caractérisée* en ce que** la face arrière de l'embase, destinée à coopérer avec la cavité glénoïde, est bombée (9), et est munie d'une quille (6) destinée à être implantée au sein de ladite cavité glénoïde.

4. Prothèse de la cavité glénoïde de la scapula selon la revendication 3, ***caractérisée* en ce que** la face arrière de l'embase (1), destinée à coopérer avec la cavité glénoïde présente deux méplats (23) situés en positions extrêmes par rapport à la partie bombée (9), et donc situés de part et d'autre de la zone bombée, destinés à prendre appui au sein de la cavité glénoïde.

5. Prothèse de la cavité glénoïde de la scapula selon l'une des revendications 3 et 4, ***caractérisée* en ce que** la quille (6) prolongeant la face arrière de l'embase est percée (7, 8), de telle sorte à favoriser la fixation secondaire de la prothèse par repousse ou migration osseuse.

6. Prothèse de la cavité glénoïde de la scapula selon l'une des revendications 1 à *5,* ***caractérisée* en ce que** les moyens assurant la coopération et la fixation de l'insert femelle (10) avec le plateau (2) de l'embase (1) sont constitués d'orifices non traversants (22) propres à coopérer avec des pions (12) de diamètre approprié issus de la partie inférieure de l'insert femelle (10), ou d'évidements réalisés à la périphérie interne et intérieure de l'embase, propres à coopérer avec des saillies de dimensions correspondantes issues de la base de l'insert femelle.

7. Prothèse de la cavité glénoïde de la scapula selon l'une des revendications 1 à 6, ***caractérisée* en ce que** les perçages (4) du plateau (2) sont chacun munis d'une rotule à expansion (24), susceptible de coopérer avec les vis d'ancrage (5), et destinée à assurer le blocage de ladite vis selon l'orientation choisie par le praticien.

8. Prothèse de la cavité glénoïde de la scapula selon l'une des revendications 1 à 7, ***caractérisée* en ce que** les vis d'ancrage (5) sont autoperforantes, autotaraudeuses et rétrocoupantes.

9. Prothèse de la cavité glénoïde de la scapula selon la revendication 7, ***caractérisée* en ce que** la rotule (24) est munie d'ergots périphériques (28) formant saillie par rapport à son enveloppe externe, lesdits ergots (28) étant destinés à être reçus dans des évidements ou logements correspondants (29), ménagés au sein d'une bague (25) soudée dans lesdits perçages (4).

10. Prothèse de la cavité glénoïde de la scapula selon l'une des revendications 6 à *9,* ***caractérisée* en ce que** l'insert femelle (10) est muni de deux pions de fixation (12), destinés à venir s'enclipser au sein des perçages (22) réalisés au sein du plateau de l'embase (1), et **en ce que** sa face inférieure est munie de dégagements (11), destinés à libérer le passage des têtes des vis d'ancrage (5).

11. Prothèse de la cavité glénoïde de la scapula selon l'une des revendications 6 à 10, ***caractérisée* en ce que** l'insert femelle (10) présente une surépaisseur au niveau de ses zones antéro-postérieures (30).

12. Prothèse de la cavité glénoïde de la scapula selon l'une des revendications 1 à 11, ***caractérisée* en ce que** les vis d'ancrage (5) sont orientables d'une valeur angulaire de plus ou moins 12 ° au sein des orifices traversants (4) du plateau (2).

## Claims

1. Prosthesis for the glenoid cavity of the scapula, comprising a seat (1), intended to be fixed to the glenoid cavity of the patient to be treated by means of anchoring screws (5), said seat (1) comprising a plate (2) able to receive a male insert (17), and said seat being able to receive a female insert (10), said inserts (10, 17) being intended to cooperate with the humeral head of the shoulder joint in question or a humeral implant fitted in the area of said joint, **characterized:**
- **in that** the outer face of the peripheral rim (3), with which the seat (1) is provided, is configured as a truncated morse cone able to cooperate with a male insert (17) provided with a bearing surface of complementary shape,
- and **in that** the plate (2) of the seat (1) is provided with means (22) intended to cooperate with a female insert (10) in order to ensure that the latter is fixed by clipping into it.

2. Prosthesis for the glenoid cavity of the scapula according to Claim 1, **characterized in that** the plate (2) is provided with two through-holes (4), each of them inclined by an angle value of between 20° and 35° relative to the general plane of the plate, and being intended to permit the passage of anchoring screws (5) into the glenoid cavity.

3. Prosthesis for the glenoid cavity of the scapula according to either of Claims 1 and 2, **characterized in that** the rear face of the seat, intended to cooperate with the glenoid cavity, is bulged (9) and is provided with a stem (6) intended to be implanted in said glenoid cavity.

4. Prosthesis for the glenoid cavity of the scapula according to Claim 3, **characterized in that** the rear face of the seat (1), intended to cooperate with the glenoid cavity, has two flat areas (23) situated at end positions relative to the bulged part (9), and thus situated on either side of the bulged zone and intended to bear inside the glenoid cavity.

5. Prosthesis for the glenoid cavity of the scapula according to either of Claims 3 and 4, **characterized in that** the stem (6) continuing the rear face of the seat is provided with holes (7, 8) in such a way as to promote secondary fixation of the prosthesis by bone growth or migration.

6. Prosthesis for the glenoid cavity of the scapula according to one of Claims 1 to 5, **characterized in that** the means ensuring the cooperation and fixation of the female insert (10) with the plate (2) of the seat (1) consist of blind holes (22) able to cooperate with pins (12) of suitable diameter issuing from the lower part of the female insert (10), or of recesses formed at the internal periphery of and inside the seat and able to cooperate with projections of corresponding dimensions issuing from the base of the female insert.

7. Prosthesis for the glenoid cavity of the scapula according to one of Claims 1 to 6, **characterized in that** the holes (4) in the plate (2) are each provided with an expansion socket (24) which is able to cooperate with the anchoring screws (5) and which is intended to immobilize said screw in the orientation chosen by the practitioner.

8. Prosthesis for the glenoid cavity of the scapula according to one of Claims 1 to 7, **characterized in that** the anchoring screws (5) are self-drilling, self-tapping and retrocutting.

9. Prosthesis for the glenoid cavity of the scapula according to Claim 7, **characterized in that** the ball (24) is provided with peripheral studs (28) projecting from its outer envelope, said studs (28) being intended to be received in corresponding recesses or indentations (29) formed in a ring (25) welded in said holes (4).

10. Prosthesis for the glenoid cavity of the scapula according to one of Claims 6 to 9, **characterized in that** the female insert (10) is provided with two fixing pins (12) intended to clip into the holes (22) formed in the plate of the seat (1), and **in that** its lower face is provided with cutouts (11) intended to permit the passage of the heads of the anchoring screws (5).

11. Prosthesis for the glenoid cavity of the scapula according to one of Claims 6 to 10, **characterized in that** the female insert (10) has an increased thickness in the area of its antero-posterior zones (30).

12. Prosthesis for the glenoid cavity of the scapula according to one of Claims 1 to 11, **characterized in that** the anchoring screws (5) can be oriented by an angle value of approximately 12° inside the through-holes (4) of the plate (2).

## Patentansprüche

1. Schulterblatt-Gelenkpfannenprothese umfassend einen Sockel (1), der dazu bestimmt ist, mit Hilfe von Verankerungsschrauben (5) an der Gelenkpfanne des prothetisch zu versorgenden Patienten befestigt zu werden, wobei der Sockel (1) eine Platte (2) aufweist, die geeignet ist, einen Einsteckeinsatz (17) aufzunehmen und wobei der Sockel geeignet ist, einen Aufnahmeinsatz (10) aufzunehmen, wobei die Einsätze (10, 17) dazu bestimmt sind, mit dem Humeruskopf des betrachteten Schultergelenks oder mit einem im Bereich des Gelenks eingesetzten Humerusimplantat zusammenzuwirken, **dadurch gekennzeichnet:**
- **daß** die Außenseite des Umfangsrandes (3), mit dem der Sockel (1) versehen ist, als Morsekegelstumpf ausgebildet ist, der geeignet ist, mit einem Einsteckeinsatz (17), welcher mit einem Bereich ergänzender Form ausgestattet ist, zusammenzuwirken, und
- **daß** die Platte (2) des Sockels (1) mit Mitteln (22) versehen ist, die dazu bestimmt sind, mit einem Aufnahmeeinsatz (10) zusammenzuwirken, um dessen Festclipsen in dieser sicherzustellen.

2. Schulterblatt-Gelenkpfannenprothese nach Anspruch 1, **dadurch gekennzeichnet, daß** die Platte (2) mit zwei durchgehenden Bohrungen (4) versehen ist, die jeweils um einen Winkel zwischen 20° und 35° gegenüber der Hauptebene der Platte geneigt sind und die dazu bestimmt sind, das Durchgreifen von Verankerungsschrauben (5) innerhalb der Gelenkpfanne zu ermöglichen.

3. Schulterblatt-Gelenkpfannenprothese nach einem der Ansprüche 1 und 2, **dadurch gekennzeichnet, daß** die Rückseite des Sockels, welche dazu bestimmt ist, mit der Gelenkpfanne zusammenzuwirken, gewölbt (9) und mit einem Kiel (6) versehen ist, der dazu bestimmt ist, innerhalb der Gelenkpfanne eingesetzt zu werden.

4. Schulterblatt-Gelenkpfannenprothese nach Anspruch 3, **dadurch gekennzeichnet, daß** die Rückseite des Sockels (1), welche dazu bestimmt ist, mit der Gelenkpfanne zusammenzuwirken, zwei Abflachungen (23) aufweist, die gegenüber dem gewölbten Teil (9) außen liegen und sich demnach auf beiden Seiten des gewölbten Bereichs befinden und die dazu bestimmt sind, sich innerhalb der Gelenkpfanne abzustützen.

5. Schulterblatt-Gelenkpfannenprothese nach einem der Ansprüche 3 und 4, **dadurch gekennzeichnet, daß** der die Rückseite des Sockels verlängernde Kiel (6) derart durchbohrt (7, 8) ist, daß die Sekundärbefestigung der Prothese durch neu gebildetes Knochenmaterial oder Knochenmigration begünstigt wird.

6. Schulterblatt-Gelenkpfannenprothese nach einem der Ansprüche 1 bis 5, **dadurch gekennzeichnet, daß** die Mittel, die das Zusammenwirken und die Befestigung des Aufnahmeeinsatzes (10) mit der Platte (2) des Sockels (1) sicherstellen, von nicht durchgehenden Öffnungen (22) gebildet sind, welche geeignet sind, mit vom unteren Teil des Aufnahmeeinsatzes (10) ausgehenden, einen entsprechenden Durchmesser aufweisenden Stiften (12) zusammenzuwirken, oder von Ausnehmungen, die am Innen- und inneren Umfang des Sockels ausgebildet und geeignet sind, mit von der Basis des Aufnahmeeinsatzes ausgehenden, entsprechende Abmessungen aufweisenden Vorsprüngen zusammenzuwirken.

7. Schulterblatt-Gelenkpfannenprothese nach einem der Ansprüche 1 bis 6, **dadurch gekennzeichnet, daß** die Bohrungen (4) der Platte (2) jeweils mit einem Spreizgelenkkopf (24) versehen sind, der geeignet ist, mit den Verankerungsschrauben (5) zusammenzuwirken, und der dazu bestimmt ist, die Festlegung der Schraube in der durch den Arzt gewählten Ausrichtung sicherzustellen.

8. Schulterblatt-Gelenkpfannenprothese nach einem der Ansprüche 1 bis 7, **dadurch gekennzeichnet, daß** die Verankerungsschrauben (5) selbstdurchbohrend, selbstschneidend und rückschneidend sind.

9. Schulterblatt-Gelenkpfannenprothese nach Anspruch 7, **dadurch gekennzeichnet, daß** der Gelenkkopf (24) mit umfangseitigen, gegenüber seinem Außenmantel vorspringenden Nasen (28) versehen ist, wobei die Nasen (28) dazu bestimmt sind, in entsprechenden Ausnehmungen oder Aufnahmen (29) aufgenommen zu werden, die in einem in die Bohrungen (4) geschweißten Ring (5) ausgebildet sind.

10. Schulterblatt-Gelenkpfannenprothese nach einem der Ansprüche 6 bis 9, **dadurch gekennzeichnet, daß** der Aufnahmeeinsatz (10) mit zwei Befestigungsstiften (12) versehen ist, die dazu bestimmt sind, in den in der Platte des Sockels (1) ausgebildeten Bohrungen (22) festgeclipst zu werden, und daß seine Unterseite mit Öffnungen (11) versehen ist, die dazu bestimmt sind, den Durchgang der Köpfe der Verankerungsschrauben (5) freizugeben.

11. Schulterblatt-Gelenkpfannenprothese nach einem der Ansprüche 6 bis 10, **dadurch gekennzeichnet, daß** der Aufnahmeeinsatz (10) im Bereich seiner anteroposterioren Bereiche eine Überdicke aufweist.

12. Schulterblatt-Gelenkpfannenprothese nach einem der Ansprüche 1 bis 11, **dadurch gekennzeichnet, daß** die Verankerungsschrauben (5) um einen Winkel von mehr oder weniger 12° innerhalb der durchgehenden Öffnungen (4) der Platte (2) ausgerichtet werden können.
